# FASCICULE DE BREVET EUROPEEN

(11) **EP 1 799 188 B1**
(45) Date de publication et mention de la délivrance du brevet: **06.05.2015**
(21) Numéro de dépôt: 05739528.7
(22) Date de dépôt: 10.03.2005
(51) Int. Cl.: A61K 38/07, A61Q 19/08, A61Q 17/04

(54) **UTILISATION DE PEPTIDES EN TANT QU'AGENT ANTIOXYDANT POUR LA PRÉPARATION D'UNE COMPOSITION COSMÉTIQUE ET/OU PHARMACEUTIQUE**
VERWENDUNG EINES PEPTIDS ALS ANTIOXIDANT IN EINER KOSMETISCHEN UND/ODER PHARMAZEUTISCHEN ZUSAMMENSETZUNG
USE OF PEPTIDES AS AN ANTIOXIDANT AGENT FOR THE PREPARATION OF A COSMETIC AND/OR PHARMACEUTICAL COMPOSITION

(30) Priorité: 12.03.2004 FR 0402582; 29.06.2004 FR 0407135
(43) Date de publication de la demande: 27.06.2007
(73) Titulaire: Ashland Specialties France, 06410 Biot (FR)
(72) Inventeur: DAL FARRA, Claude, F-06650 Opio (FR); DOMLOGE, Nouha, F-06560 Valbonne (FR); PEYRONEL, Dominique, F-13014 Marseille (FR)
(74) Mandataire: Macquet, Christophe
(86) Numéro de dépôt international: PCT/FR2005/000569
(87) Numéro de publication internationale: WO 2005/097060

(56) Documents cités:
- US-A- 5 028 419
- PATENT ABSTRACTS OF JAPAN vol. 1995, no. 03, 28 avril 1995 (1995-04-28) -& JP 06 345797 A (YAKULT HONSHA CO LTD), 20 décembre 1994 (1994-12-20)
- PATENT ABSTRACTS OF JAPAN vol. 2000, no. 01, 31 janvier 2000 (2000-01-31) & JP 11 292897 A (SUETSUNA YOKO), 26 octobre 1999 (1999-10-26)
- DEL CORSO A. ET AL.: "Physiological Thios as Promoters of Glutathione Oxidation and Modifying Agents in Protein S-Thiolation" ARCHIVES OFBIOCHEMISTRY AND BIOPHYSICS, vol. 397, no. 2, 15 janvier 2002 (2002-01-15), pages 392-398, XP002312958
- ASKEW S C ET AL: "Chemical mechanisms underlying the vasodilator and platelet anti-aggregating properties of S-nitroso-N-acetyl-DL-penicillamine and S-nitrosoglutathione" BIOORGANIC & MEDICINAL CHEMISTRY, ELSEVIER SCIENCE LTD, GB, vol. 3, no. 1, janvier 1995 (1995-01), pages 1-9, XP002225525 ISSN: 0968-0896

## Description

L'invention concerne le domaine de la cosmétique et de la pharmaceutique, notamment le domaine de la dermatologie. La présente invention a pour objet l'utilisation d'un peptide Cys-Gly dimérisé par l'intermédiaire d'un pont disulfure, en tant qu'agent antioxydant, dans ou pour la préparation d'une composition cosmétique et/ou dermatologique et/ou pharmaceutique. Ledit peptide ou la composition le contenant étant, entre autres, destinés à prévenir ou traiter les dommages cellulaires provoqués par les radicaux libres induits, notamment, par les polluants atmosphériques et/ou par le rayonnement ultraviolet.

Le vieillissement des organismes et des organes, tels que la peau, est un processus évident mais aussi extrêmement complexe qui peut prendre des formes variées d'un individu à un autre. Le vieillissement de la peau n'est qu'un seul aspect du vieillissement global de l'organisme. Divers facteurs interviennent dans ce processus, des facteurs d'origines endogènes bien sûr, mais aussi des facteurs d'origines exogènes. Ainsi, une exposition excessive aux rayonnements solaires et, notamment, aux rayons ultraviolets, est un phénomène qui accélère le vieillissement, on parle d'ailleurs d'un vieillissement photo-induit. On assiste ainsi à un déclin prématuré de la quantité et de la qualité des constituants de la peau et de l'épiderme en particulier. En effet, l'action de la lumière et, notamment, des rayonnements UV entraînent la formation de radicaux libres. D'autres facteurs d'origine extérieure, tels que la pollution et les agressions de toutes sortes peuvent agir de façon néfaste sur la peau. On sait, par exemple, que la toxicité des polluants atmosphériques, notamment des polluants gazeux tels que le dioxyde de souffre, l'ozone et les oxydes d'azote, est liée en grande partie à leur pouvoir initiateur de radicaux libres. Les cellules de la peau, en contact direct avec le milieu extérieur, sont donc particulièrement exposées à ces polluants.

Les radicaux libres sont des sources de phénomènes d'oxydation. Ce sont des constituants chimiques très réactionnels qui interviennent de manière transitoire dans de nombreux mécanismes cellulaires. Les radicaux libres photo-induits proviennent en grande partie de l'oxygène moléculaire. Etant donné son abondance dans l'organisme et sa capacité à accepter les électrons, les radicaux libres qui en dérivent sont les plus nombreux à participer aux réactions radicalaires. On peut ainsi dénombrer parmi ces espèces réactives : l'oxygène singulet (produit de l'excitation de l'oxygène moléculaire par les photons) ; le radical anion superoxyde (produit de l'addition d'un électron sur l'oxygène moléculaire) ; le peroxyde d'hydrogène (non radicalaire mais qui peut donner lieu à la production de radicaux hydroxyles) ; le radical hydroxyle (très oxydant donc très réactif et très toxique pour les cellules).

Ces radicaux libres, s'ils sont incontrôlés, peuvent rapidement réagir avec des molécules avoisinantes donnant naissance à des produits toxiques pouvant interférer avec le fonctionnement physiologique normal des cellules. Par les phénomènes d'oxydation et les attaques radicalaires qu'ils engendrent, les radicaux libres vont produire un stress oxydant et provoquer des dégâts importants. Ils vont, par exemple, provoquer des dommages au niveau des membranes cellulaires ; ils vont altérer des macromolécules (péroxydation lipidique, carbonylation des protéines) et engendrer des mutations et des ruptures au niveau des brins d'ADN, ce qui peut conduire à la mort de la cellule. Ils sont ainsi souvent associés aux phénomènes de nécrose des tissus. La présence de ces radicaux libres dans la peau est probablement responsable d'un grand nombre d'effets indésirables. Par exemple, le vieillissement est observé prématurément au niveau de la peau comme une conséquence du photo-vieillissement, accélérant entre autres les phénomènes de détérioration de l'élastine, du collagène ou de la fibronectine. Ces dommages peuvent parfois aboutir à des processus de cancérisation. Il est donc important de mettre au point des systèmes capables de lutter activement contre ces réactifs et leurs conséquences.

L'organisme possède des mécanismes enzymatiques de défense contre ces radicaux libres, trois enzymes constituent les clés de voûte de cette protection : la famille des superoxydes dismutases (SOD) qui dismutent l'ion superoxyde en peroxyde d'hydrogène; les catalases, généralement confinées dans les peroxisomes, accélérant la réaction spontanée qui transforme le peroxyde d'hydrogène en oxygène et en eau ; et la glutathion peroxydase, enzyme cytosolique séléniée réduisant le peroxyde d'hydrogène en présence de glutathion. Cependant ces systèmes de défenses antioxydantes s'avèrent souvent insuffisants devant les nombreux stress et agressions extérieures auxquels sont soumis les organismes et la peau en particulier.

De nombreux actifs ont déjà été mis au point afin de combattre activement ces radicaux libres et les réactions d'oxydation qu'ils engendrent. On peut notamment citer, par exemple, les vitamines A, C ou E, les polyphénols (notamment ceux d'origine végétale), ou encore la quinoléine et ses dérivés- Cependant, ces actifs ne permettent pas de résoudre ces problèmes de manière réellement satisfaisante, et de nombreux progrès restent encore à faire afin de pouvoir disposer d'actifs présentant des propriétés véritablement convenables. De nombreux acides aminés ont aussi été utilisés en tant qu'agent antioxydant. Par exemple, la demande de brevet JP06345797A décrit une composition cosmétique comprenant un dipeptide de formule H-Cys-X-OH, la demande de brevet JP11292897 décrit le pentapeptide Val-Pro-Cys-Gly-Lys comme antioxydant, la demande de brevet US 5 028 419 décrit des peptides Cys-X-Y-Cys comme antioxydants, et Del Corso et al. (Archives of Biochemistry and Biophysics, vol.397, n°2, pp392-398) montrent que le peptide Cys-Gly améliore l'action antioxydante du glutathion. Cependant, ils présentent le désavantage d'être très rapidement métabolisés dans les cellules et possèdent ainsi une très faible efficacité. Il subsiste donc toujours un besoin d'actif ayant une action réellement efficace en tant qu'agent antioxydant et/ou en tant qu'agent anti-radicalaire,

La présente invention vise à combler cette lacune et a pour principal objectif de fournir un nouveau principe actif antioxydant et/ou anti-radicalaire. Les inventeurs ont réussi à sélectionner des substances particulières présentant des propriétés remarquables lorsque celles-ci sont appliquées sur la peau. Ils ont ainsi découvert, de manière inattendue, qu'un peptide correspondant à la formule générale (AA)n-Cys-Gly-(AA)n, et en particulier le peptide de séquence Cys-Gly dimérisé par l'intermédiaire d'un pont disulfure, possède des propriétés antioxydantes lorsque celui-ci est appliqué sur la peau. Ce peptide de faible taille moléculaire possède, entre autres, comme particularité une très grande stabilité dans les cellules. Ce peptide, par opposition aux acides aminés qui le constituent, est très peu dégradé, ce qui lui permet d'agir d'une manière particulièrement efficace.

Ainsi, selon un premier aspect, la présente invention a pour objet l'utilisation d'une quantité efficace d'au moins un peptide correspondant à la formule générale (I):

(Cys-Gly (I)

en tant qu'agent antioxydant et/ou anti-radicalaire, seul ou en association avec au moins un autre agent actif, dans ou pour la préparation d'une composition cosmétique et/ou dermatologique, le peptide étant dimérisé par l'intermédiaire d'un pont disulfure.

Ainsi, le peptide selon l'invention est un dipeptide sous forme dimérisé, c'est-à-dire correspondant à la formule générale Gly-Cys-S-S-Cys-Gly. Ce dipeptide dimérisé, de séquence Cys-Gly, sera avantageusement utilisé en tant qu'agent actif, seul ou en association avec au moins un autre agent actif, dans ou pour la préparation d'une composition cosmétique et/ou dermatologique.

On entend par « dipeptide de séquence Cys-Gly dimérisé », deux dipeptides reliés entre eux par des ponts disulfures ; les acides aminés cystéine, composant le dipeptide, possédant des fonctions thiols capables de créer ce type de liaison. Ce composé possède donc la séquence décrite plus haut, il est appelé diglycylcystine : les deux résidus cystéines, liés entre eux par un groupement thiol, possédant le nom de cystine.

Le terme "acide aminé" se réfère ici à tout acide organique naturel ou non naturel ayant la formule (II) :

-NHR-CR-C(O)-O- (II)

où chaque -R est indépendamment sélectionné entre un hydrogène et un groupement alkyl ayant entre 1 et 12 atomes de carbone. Préférentiellement, au moins un groupement -R de chaque acide aminé est un hydrogène. Par le terme "alkyl", on entend ici une chaîne carbonée pouvant être linéaire ou ramifiée, substituée (mono- ou poly-) ou non-substituée ; saturée, mono-saturée (une double ou triple liaison dans la chaîne) ou poly-insaturée (deux ou plusieurs doubles liaisons, deux ou plusieurs triples liaisons, une ou plusieurs doubles liaisons et une ou plusieurs triples liaisons dans la chaîne).

Le terme « peptide » désigne un enchaînement de deux ou plusieurs acides aminés liés entre eux par des liaisons peptidiques ou par des liaisons peptidiques modifiées.

Par peptide, il faut entendre le peptide naturel ou synthétique de l'invention tel que décrit ci-dessus, qu'il soit obtenu par protéolyse ou de manière synthétique ou encore tout peptide naturel ou synthétique dont la séquence est totalement ou partiellement constituée par la séquence du peptide précédemment décrit.

Il se peut que pour des questions de résistance à la dégradation, il soit nécessaire d'utiliser une forme protégée du peptide selon l'invention. La forme de protection doit évidemment être une forme biologiquement compatible et doit être compatible avec une utilisation dans le domaine des cosmétiques ou de la pharmacie. De nombreuses formes de protection biologiquement compatibles peuvent être envisagées, elles sont bien connues de l'homme du métier comme, par exemple, l'acylation ou l'acétylation de l'extrémité amino-terminale, ou l'amidation ou l'estérification de l'extrémité carboxy-terminale. Ainsi, l'invention concerne une utilisation telle que définie précédemment caractérisée par le fait que le peptide est sous forme protégée ou non. De préférence, on utilise une protection basée soit sur l'acylation ou l'acétylation de l'extrémité amino-terminale, soit sur l'amidation ou l'estérification de l'extrémité carboxy-terminale, soit encore des deux.

Les dérivés d'acides aminés et les dérivés de peptides peuvent aussi constituer le peptide selon l'invention. Ces dérivés concernent, par exemple, les acides aminés et les peptides reliés entre eux par une liaison pseudo-peptidique. On entend par "liaison pseudo-peptidique" ainsi que tous types de liaisons susceptibles de remplacer les liaisons peptidiques "classiques".

Dans le domaine des acides aminés, la géométrie des molécules est telle qu'elles peuvent théoriquement se présenter sous la forme d'isomères optiques différents. Il existe, en effet, une conformation moléculaire de l'acide aminé (AA) telle qu'elle dévie à droite le plan de polarisation de la lumière (conformation dextrogyre ou D-aa), et une conformation moléculaire de l'acide aminé (aa) telle qu'elle dévie à gauche le plan de polarisation de la lumière (conformation lévogyre ou L-aa). La nature n'a retenu pour les acides aminés naturels que la conformation lévogyre. En conséquence, un peptide d'origine naturelle ne sera constitué que d'acides aminés de type L-aa. Cependant la synthèse chimique en laboratoire permet de préparer des acides aminés ayant les deux conformations possibles. A partir de ce matériel de base, il est ainsi possible d'incorporer lors de la synthèse de peptide aussi bien des acides aminés sous forme d'isomères optiques dextrogyre ou lévogyre. Ainsi, les acides aminés constituant le peptide selon l'invention peuvent être sous configuration L- et D- ; de manière préférentielle, les acides aminés sont sous forme L. Le peptide selon l'invention peut donc être sous forme L-, D-ou DL-.

Les peptides selon l'invention peuvent être préparés par toutes méthodes appropriées, ainsi les peptides peuvent être des peptides isolés à partir de peptides et de protéines existant naturellement, des peptides recombinants, des peptides de synthèse, ou encore des peptides produits par une combinaison de ces méthodes. Bien entendu, les méthodes afin de préparer les peptides selon l'invention sont bien connues de l'homme du métier. Ainsi, le peptide selon l'invention peut être un peptide d'origine naturelle ou synthétique. Préférentiellement selon l'invention, le peptide est obtenu par synthèse chimique.

Un aspect essentiel de l'invention est l'utilisation d'au moins un peptide tel que défini précédemment, dans ou pour la préparation d'une composition cosmétique et/ou pharmaceutique, à usage topique, destinée, notamment, à obtenir une activité protectrice vis-à-vis des espèces réactives de l'oxygène. Lesdits peptides étant avantageusement utilisés en tant qu'agent antioxydant et/ou en tant qu'agent anti-radicalaire. Par agent anti-radicalaire, on entend tout composé capable de piéger les radicaux libres. Cet actif est, en effet, capable de bloquer les réactions en chaînes des radicaux libres avant les étapes ultimes de dégradation des constituants biologiques de la peau, on parle alors de composés antioxydants.

Le peptide selon l'invention peut être avantageusement utilisé dans ou pour la préparation d'une composition cosmétique et/ou pharmaceutique, en tant qu'agent photo-protecteur et, plus particulièrement, en tant qu'agent photo-protecteur dit « secondaire ». On distingue, en effet, les agents photo-protecteurs primaires des agents photo-protecteurs secondaires. Les agents photo-protecteurs primaires sont des substances qui exercent un pouvoir physique : ils sont en mesure d'absorber les rayonnements UV et de les restituer sous forme de chaleur afin de protéger la peau. Les agents photo-protecteurs secondaires sont des substances qui ont plutôt un effet biologique ; ce sont, par exemple, les agents de type antioxydant qui interrompent les chaînes de réactions photo-chimiques qui sont déclenchées lorsque le rayonnement UV pénètre dans la peau.

Par ses activités particulières, les composés selon l'invention pourront être utilisés avantageusement dans ou pour la préparation d'une composition cosmétique et/ou pharmaceutique, à usage topique, destinée, notamment, à lutter de manière préventive et/ou curative contre les manifestations du vieillissement cutané et, tout particulièrement, afin de lutter contre et/ou de prévenir le vieillissement photo-induit. Par manifestations cutanées du vieillissement on entend toutes modifications de l'aspect extérieur de la peau dues au vieillissement comme, par exemple, les rides et ridules, la peau flétrie, la peau molle, la peau amincie, le manque d'élasticité et/ou de tonus de la peau, la peau terne et sans éclat ou les taches de pigmentation de la peau, mais également toutes modifications internes de la peau qui ne se traduisent pas systématiquement par un aspect extérieur modifié comme, par exemple, toutes dégradations internes de la peau consécutives à une exposition aux rayonnements ultra-violets. Le peptide selon l'invention ou la composition le contenant permettra de lutter, en particulier, contre la perte d'élasticité et de fermeté de la peau.

Le peptide selon l'invention permet de protéger la peau contre tous types d'agressions extérieures. Ainsi il peut être destiné à protéger les substrats kératiniques et, plus particulièrement, à protéger la peau et/ou les phanères contre tous les types d'agressions extérieures. L'utilisation de ce composé, ou d'une composition le contenant, va permettre aux substrats kératiniques d'être protégés et de mieux résister au stress que produit l'environnement.

On entend, par le terme "agression extérieure", les agressions que peut produire l'environnement. A titre d'exemple, on peut citer des agressions telles que la pollution, les UV, ou encore les produits à caractère irritant tels que les tensioactifs, les conservateurs ou les parfums. Par pollution, on entend aussi bien la pollution « extérieure », due par exemple aux particules de diesel, à l'ozone ou aux métaux lourds, que la pollution « intérieure » qui peut être due notamment aux émissions de solvants de peintures, de colles, ou de papier-peints (tels que toluène, styrène, xylène ou benzaldehydes), ou bien encore la fumée de cigarette.

Le peptide selon l'invention permettra tout particulièrement de protéger la peau et/ou les phanères des agressions qui aboutiraient à la formation de radicaux libres et créeraient ainsi un stress oxydatif. Ainsi, le composé selon l'invention permettra de lutter contre les dommages esthétiques provoqués sur la peau et/ou les cheveux par les radicaux libres induits, notamment, par les polluants atmosphériques et par le rayonnement. Le composé pourra être utilisé dans ou pour la préparation d'une composition cosmétique et/ou pharmaceutique destinée à prévenir ou traiter les dommages cellulaires provoqués par les radicaux libres induits, entre autres, par les polluants atmosphériques et/ou par le rayonnement ultraviolet. Plus généralement, par son action antioxydante, le composé selon l'invention pourra être utilisé dans ou pour la fabrication d'une composition destinée à protéger la peau contre le stress, notamment contre le stress oxydatif.

Par ailleurs, selon un autre aspect, le peptide selon l'invention permettra avantageusement de lutter contre les manifestations de l'inflammation résultant de ces agressions ; il permettra notamment de lutter contre les manifestations cutanées de l'inflammation.

Selon un autre aspect, l'invention concerne une composition cosmétique et/ou dermatologique caractérisée en ce qu'elle contient, dans un milieu acceptable, comme principe actif, au moins un peptide tel que défini précédemment. Selon une méthode de réalisation particulièrement avantageuse de l'invention, la composition contient un peptide de séquence Cys-Gly. Selon une méthode de réalisation actuellement préférée de l'invention, le dipeptide sera sous forme dimérisé. Il est bien entendu que le peptide selon l'invention peut être utilisé seul ou en association avec au moins un autre agent actif.

La composition contenant le peptide selon l'invention peut être une composition cosmétique ou dermatologique ou pharmaceutique. Préférentiellement selon l'invention, la composition est une composition cosmétique car elle est destinée à améliorer l'aspect et les performances cutanées générales de l'individu qui en fait usage. La composition selon l'invention est préférentiellement une composition cosmétique et/ou dermatologique adaptée à l'administration par voie topique cutanée comprenant un milieu cosmétiquement ou pharmaceutiquement acceptable. L'invention concerne, en particulier, une composition cosmétique destinée à obtenir une action antioxydante et/ou une action anti-radicalaire lorsqu'elle est appliquée sur la peau.

Il est bien évident que l'invention s'adresse aux mammifères en général et plus particulièrement aux êtres humains.

Selon un mode de réalisation avantageux de l'invention, les composés précités selon l'invention sont, préalablement à leur utilisation, solubilisés dans un ou plusieurs solvants cosmétiquement ou pharmaceutiquement acceptables, classiquement utilisés par l'homme du métier, comme l'eau, l'éthanol, le propylène glycol, le butylène glycol, le dipropylène glycol, les diglycols éthoxylés ou propoxylés, les polyols cycliques, la vaseline, une huile végétale ou tout mélange de ces solvants. Selon encore un autre mode de réalisation avantageux de l'invention, les composés précités sont, préalablement à leur utilisation, solubilisés dans un vecteur cosmétique ou pharmaceutique comme les liposomes ou adsorbés sur des polymères organiques poudreux, des supports minéraux comme les talcs et bentonites, et plus généralement solubilisés dans, ou fixés sur, tout vecteur cosmétiquement ou pharmaceutiquement acceptable.

Il est bien entendu évident que le composé selon l'invention peut être utilisé seul ou bien en association avec au moins un autre agent actif, dans ou pour la préparation d'une composition cosmétique et/ou dermatologique et/ou pharmaceutique.

La quantité efficace de principe actif correspond à la quantité nécessaire afin d'obtenir le résultat désiré. Selon un mode de réalisation avantageux de l'invention, le peptide précité est présent dans les compositions de l'invention à une concentration comprise entre 0,005 et 500 ppm (parties par million) environ, et préférentiellement à une concentration comprise entre 0,05 et 50 ppm, environ, par rapport au poids total de la composition finale. Selon un mode de réalisation tout particulièrement avantageux, la concentration de l'actif selon l'invention est comprise entre 0,1 et 5 ppm, environ, par rapport au poids total de la composition finale.

Quelle que soit la forme de l'invention, la composition selon l'invention peut être ingérée, injectée ou appliquée sur la peau (sur toute zone cutanée du corps), les cheveux, les ongles ou les muqueuses. Selon le mode d'administration, la composition selon l'invention peut se présenter sous toutes les formes galéniques normalement utilisées. Préférentiellement, les compositions selon la présente invention se présenteront sous une forme galénique adaptée à l'administration par voie topique cutanée. Elles couvrent toutes les formes cosmétiques ou dermatologiques. Ces compositions doivent donc contenir un milieu cosmétiquement acceptable, c'est-à-dire compatible avec la peau, les poils ou les cheveux.

Ces compositions peuvent notamment se présenter sous forme d'une solution aqueuse, hydroalcoolique ou huileuse ; d'une émulsion huile-dans-eau, eau-dans-huile ou émulsions multiples ; elles peuvent aussi se présenter sous forme de crèmes, de suspensions ou encore de poudres, adaptées à une application sur la peau, les muqueuses, les lèvres et/ou les cheveux.

Ces compositions peuvent être plus ou moins fluides et avoir l'aspect d'une crème, d'une lotion, d'un lait, d'un sérum, d'une pommade, d'un shampooing, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau. Pour l'injection, la composition selon l'invention peut se présenter sous forme de lotion aqueuse, huileuse ou sous forme de sérum. Pour l'application sur les yeux, la composition peut se présenter sous forme de gouttes et pour l'ingestion, elle peut se présenter sous forme de capsules, de granulés, de sirops ou de comprimés. Ces compositions comprennent, en outre, tout additif usuellement utilisé dans le domaine d'application envisagé ainsi que les adjuvants nécessaires à leur formulation, tels que des solvants, des épaississants, des diluants, des antioxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

Dans tous les cas, l'homme du métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre de 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition. Bien entendu, l'homme du métier veillera à choisir les éventuels composés complémentaires, actifs ou non-actifs, et/ou leurs quantités, de telle sorte que les propriétés avantageuses du mélange ne soient pas, ou sensiblement pas, altérées par l'adjonction envisagée.

Les compositions selon l'invention trouvent une application notamment comme compositions cosmétiques ou pharmaceutiques pour la peau, les muqueuses et/ou les semi-muqueuses, mais aussi comme composition cosmétique pour les cheveux et/ou les poils. Elles trouvent une application toute particulière en tant que produit de protection et/ou de soin de la peau. La composition selon l'invention est, préférentiellement, une composition capable d'entretenir ou de soigner la peau, mais c'est aussi une composition apte à prévenir et/ou à lutter contre les manifestations du vieillissement cutané, particulièrement contre le vieillissement photo-induit. La composition selon l'invention est, de la même manière, capable de protéger la peau contre les agressions extérieures provoquées, notamment, par l'action du rayonnement solaire ou par d'autres agents physiques, chimiques ou biologiques, en agissant principalement par suppression de la formation de radicaux libres oxygénés et des réactions qu'ils entraînent.

On peut également envisager une application dans le domaine des compositions de maquillage de la peau du visage et du corps, tels que les rouges à lèvres, les fonds de teint, les crèmes teintées, les sticks anti-cernes, les compositions anti-solaires ou de bronzage artificiel.

Les compositions, objet de l'invention, trouvent leur application dans grand nombre de traitements notamment cosmétiques ou dermatologiques, et elles peuvent constituer une composition cosmétique, notamment pour le traitement, la protection, le soin, le démaquillage et/ou le nettoyage de la peau, des lèvres et/ou des cheveux, et/ou pour le maquillage de la peau, des lèvres, des cils et/ou du corps. La composition selon l'invention peut également consister en des préparations solides comprenant également des savons ou des pains de nettoyage. La composition peut être aussi conditionnée sous forme d'une composition pour aérosol comprenant également un agent propulseur sous pression. La composition peut être aussi à usage bucco-dentaire, comme par exemple une pâte de dentifrice. La composition de l'invention peut aussi être une composition cosmétique destinée à une administration par voie orale. Pour une administration par voie orale, la composition selon l'invention peut se présenter sous toutes formes adaptées, particulièrement sous forme d'une solution buvable, d'un sirop, d'un comprimé, d'une dragée, d'une gélule ou encore d'un aliment ou complément nutritionnel.

Selon un autre aspect, la présente invention concerne un procédé de traitement cosmétique pour traiter les peaux, notamment les peaux âgées et/ou pour combattre les phénomènes de vieillissement cellulaire consistant à appliquer sur la surface de la peau une quantité efficace de la composition telle que définie précédemment afin obtenir l'action désirée. Selon une méthode de réalisation préférée de l'invention, le procédé selon l'invention permet de lutter contre le vieillissement photo-induit.

La présente invention concerne, de la même manière, un procédé de traitement cosmétique afin de protéger la peau et/ou les phanères contre tous types d'agressions extérieures. La présente invention concerne, de la même manière, un procédé de traitement cosmétique permettant de lutter contre les dommages inesthétiques provoqués sur la peau et les cheveux par les radicaux libres induits notamment par les polluants atmosphériques et le rayonnement ultraviolet. Ce procédé consistant à appliquer sur la peau ou les cheveux une composition telle que définie précédemment. La présente invention est aussi relative à un procédé de traitement de l'inflammation cutanée consistant à appliquer sur la peau ou les cheveux une composition telle que définie précédemment.

Des modes de réalisation particuliers de ce procédé de traitement cosmétique résultent également de la description précédente. Le procédé de traitement cosmétique de l'invention peut être mis en oeuvre notamment en appliquant les compositions cosmétiques telles que défines ci-dessus, selon la technique d'utilisation habituelle de ces compositions, par exemple : application de crèmes, de gels, de sérums, de lotions, de laits, de shampooings ou de compositions anti-solaires, sur la peau ou sur les cheveux, ou encore, application de dentifrice sur les gencives.

D'autres avantages et caractéristiques de l'invention apparaîtront mieux à la lecture des exemples donnés à titre illustratif et non limitatif.

### Exemple 1 : Mise en évidence de l'effet anti-oxydant du peptide Cys-Gly dimérisé sur des fibroblastes.

### I. Protocole expérimental :

Des fibroblastes primaires, à un passage compris entre P6 et P13, ont été ensemencés sur des boîtes de diamètres 100 à environ 250 000 cellules par boite. Ces cellules sont ensuite incubées à 37°C, dans une atmosphère saturée à 5 % de CO₂, jusqu'à ce qu'elles atteignent 60 à 70 % de confluence.

Une fois la confluence désirée atteinte, les cellules reçoivent l'actif, ou du milieu de culture, pendant une durée de 24 heures. Ces cellules sont ensuite irradiées, ou non, par des UVB à 100 mJ/cm² (ou aux UVA). Pendant le temps d'irradiation, les boîtes sont traitées avec du PBS seul ou avec le produit à tester dilué dans du PBS.

Quatre conditions d'étude ont ainsi été réalisées : deux conditions contrôles avec du milieu de culture et avec, ou sans, irradiation aux UVB (ou aux UVA); deux conditions avec l'actif (le dipeptide dimérisé Cys-Gly selon l'invention, dilué à 1 % dans du milieu de culture) et avec, ou sans, irradiation aux UVB (ou aux UVA). Les cellules sont traitées différemment suivant les tests effectués ultérieurement :
- Dans le cas du dosage de l'activité catalase, les cellules sont remises en culture pendant 30 minutes avec ou sans la présence de l'actif selon l'invention.
- Pour le dosage de la carbonylation des protéines, les cellules sont directement arrêtées après l'irradiation.
- Pour la révélation de l'activité SOD, les cellules sont remises en culture pendant 24 heures, en présence ou non, de l'actif selon l'invention.
- Pour le dosage de la peroxydation lipidique, les cellules sont traitées avec des UVA.

Les échantillons à tester sont préparés en récupérant toutes les cellules des diamètres 100 et en les lysant avec un tampon adéquat contenant des inhibiteurs de protéases ainsi qu'en les « scrappant ». La suspension cellulaire obtenue est alors broyée (par passage à la seringue) afin d'extraire le plus de protéines possible. Puis, finalement, les extraits protéiques sont centrifugés à 12500 rpm durant 5 min pour ne récupérer que le surnageant. Un dosage de protéines par BCA est effectué afin de standardiser tous les échantillons à la même concentration.

### II. Mise en évidence de l'activité de la Super Oxyde Dismutase :

La Super Oxyde Dismutase (SOD) est un système enzymatique qui assure une protection efficace contre les radicaux libres. Cette enzyme catalyse en effet la dismutation d'un radical libre, l'anion superoxyde, en peroxyde d'hydrogène. Ce peroxyde d'hydrogène sera éliminé par la catalase.

Le principe de ce test est basé sur une migration des protéines totales d'un extrait cellulaire, sur un gel de polyacrylamide (gel page natif), qui sera ensuite coloré afin de permettre la mise en évidence de l'activité de la SOD.

Les échantillons à tester, correspondant aux quatre conditions décrites précédemment, sont déposés sur un gel de polyacrylamide à 8 % et soumis à une électrophorèse durant 2 heures à 100 V. A la fin de la migration, le gel est coloré par un mélange de NBT (Nitrobleu de tetrazolium) et de riboflavine. Pour ce test, les échantillons ont reçu une application de l'actif selon l'invention à 1 % pendant l'irradiation aux UVB à 100 mJ/cm² et 24 heures avant et après le stress.

L'absence de coloration démontre la présence d'une activité SOD. La riboflavine, en présence de lumière, forme un anion superoxyde pris en charge par le NBT et résultant en une coloration du gel en violet. En présence de la SOD, l'anion superoxyde sera dégradé, et la coloration violette ne se fera donc pas. Les bandes achromatiques obtenues peuvent ensuite être quantifiées par rapport à leur intensité.

Le tableau ci-dessous illustre le résultat de la quantification de l'intensité des bandes, résultant de l'activité de la SOD, en fonction des différentes conditions d'étude.

| **Conditions d'étude** | **% d'intensité des bandes Achromatiques** |
|---|---|
| - actif / - UVB | 100 |
| - actif / + UVB | 146,15 |
| + actif / - UVB | 123,08 |
| + actif / + UVB | 292,31 |

Les résultats obtenus démontrent qu'en présence de l'actif selon l'invention l'activité de la SOD augmente. L'intensité des bandes, représentative de l'activité de la SOD, double lorsque l'actif est appliqué sur des cellules ayant subi une irradiation aux UVB, par rapport aux cellules n'ayant pas reçu l'actif.

### III. Dosage de l'activité Catalase :

La Catalase est une des enzymes les plus importantes intervenant dans le système de défense contre les radicaux libres. C'est un puissant antioxydant cellulaire. Elle agit en réduisant le peroxyde d'hydrogène (H₂0₂), produit par la SOD, en oxygène et en eau (O₂ et H₂O). L'étude de l'activité de la Catalase s'effectue par des mesures spectrophotométriques. L'activité, présente dans l'échantillon, est calculée en mesurant la vitesse de disparition du peroxyde d'hydrogène.

Lors de leur préparation, les échantillons à tester sont traités avec le dipeptide selon l'invention, à 1 %, 24 heures avant l'irradiation, pendant l'irradiation aux UVB à 100 mJ/cm², et encore 30 minutes après l'irradiation. La cinétique de disparition de l'H₂O₂ dans l'échantillon, dilué dans un tampon phosphate, est mesurée à une longueur d'onde de 240 nm ; le zéro aura préalablement été effectué avec un tampon phosphate à 50 mM, puis la concentration du substrat (H₂O₂), dans ce même tampon, aura été ajustée à 14 mM, ce qui correspond à une absorbance comprise entre 0,52 et 0,55.

Cette étude met en évidence une augmentation de l'activité de la catalase lorsque les cellules sont traitées avec l'actif par comparaison avec les cellules non traitées.

### IV. Dosage de la carbonylation des protéines :

La carbonylation des protéines est un phénomène résultant du stress oxydant et de ses conséquences : l'oxydation des macromolécules par les radicaux libres. Cette carbonylation provient du clivage oxydatif des protéines ou d'une oxydation des résidus arginine, lysine, proline ou thréonine. Le dosage de la carbonylation des protéines s'effectue grâce à une technique EIA « Enzyme Immuno Assay ».

La première étape de cette méthode consiste à complexer le DNP avec les échantillons, d'une part, et d'autre part, avec une gamme de BSA oxydée. Ce réactif se fixe spécifiquement sur les groupements carbonyls qui seront dosés par méthode ELISA, grâce à un anticorps anti-DNP couplé à une peroxydase. La gamme de BSA oxydée (dont on connaît la concentration en groupements carbonyls) est utilisée pour l'étalonnage. Pour cette manipulation, les échantillons ont été mis en présence de l'actif selon l'invention à 1 %, 24 heures avant et pendant l'irradiation aux UVB à 100 mJ/cm².

Une fois les échantillons et la gamme complexés avec le réactif DNP, ils sont déposés sur une plaque 96 puits pendant toute une nuit, puis chaque puits est saturé avec de la BSA réduite. Les puits sont, par la suite, lavés et marqués par l'anticorps anti-DNP biotinylé. Un nouveau lavage est effectué afin de coupler le complexe streptavidine-peroxidase avec la biotine. Après un dernier lavage, le substrat de l'enzyme, le TMB, est ajouté dans chaque puits. La lecture est effectuée à 490 nm après addition d'acide sulfurique 2,5 M. La concentration en groupement carbonyl de chaque échantillon est déterminée grâce à la gamme de BSA oxydée.

Le tableau ci-dessous illustre la concentration en groupement carbonyl (en µmol/g de protéines) pour chaque échantillon en fonction des différentes conditions étudiées.

| **Conditions d'étude** | **Concentration en gpt carbonyl (µmol/g de prot.)** |
|---|---|
| -actif / - UVB | 2,31 |
| + actif / - UVB | 1,92 |
| - actif / + UVB | 2,57 |
| + actif / + UVB | 1,90 |

Les résultats obtenus démontrent une diminution de la carbonylation des protéines lorsque les cellules sont traitées avec l'actif selon l'invention. On observe plus particulièrement une importante diminution de cette carbonylation lorsque les cellules sont traitées avec l'actif et soumises à une irradiation, par comparaison aux cellules irradiées mais non traitées avec l'actif.

### V. Dosage de la peroxydation lipidique :

Parmi les conséquences néfastes majeures causées par le stress oxydatif, on peut citer les dommages provoqués aux macromolécules telles les lipides et, plus particulièrement, les phénomènes de péroxydation des lipides. Cette péroxydation est le résultat d'un mécanisme d'addition de radicaux libres sur les acides gras causant ainsi des fragmentations ou des altérations de sa structure. Le dosage de la peroxydation lipidique permet donc de quantifier les dégâts causés par le stress oxydatif et/ou de quantifier la protection contre ce stress.

Le principe de ce dosage est basé sur la formation d'un chromogène de couleur rose absorbant à 532 nm. Ce composé est formé par l'association, en milieu acide et à chaud, d'une molécule de malonedialdéhide (MDA), produite par péroxydation lipidique, avec deux molécules d'acides thiobarbiturique (TBA). La quantité de MDA produite est déterminée par une lecture au spectrophotomètre ; cette quantité de MDA est proportionnelle au phénomène de péroxydation lipidique.

Le dosage de la peroxydation lipidique a été effectué sur les cellules irradiées (ou non) avec 5 J/cm² d'UVA. Le tableau ci-dessous illustre le résultat des mesures de la quantité de MDA obtenue en fonction des différentes conditions d'étude.

| **Conditions d'étude** | Quantité de MDA (pg/mg de protéines) | % d'inhibition par rapport aux conditions contrôles |
|---|---|---|
| **a. CTR** | 743 | - |
| **b. Act** | 673 | - 13,45 % |
| **c. UVA CTR** | 955 | - |
| **d. UVA Act** | 627 | - 34,34 % |

Cette étude met en évidence une inhibition du phénomène de péroxydation lipidique lorsque les cellules sont traitées avec l'actif par comparaison avec les cellules non traitées. Cette inhibition est encore plus significative lorsque les cellules ont subit un traitement avec des UVA.

### VI. Conclusion

La SOD et la catalase sont des systèmes enzymatiques spécialisés dans la protection des cellules contre les dommages occasionnés par les radicaux libres. Ainsi, une augmentation de l'activité de la SOD et de la catalase résulte d'une activation du système de protection cellulaire existant naturellement, cette activation permettant une meilleure protection contre les stress oxydants. Ces tests nous permettent donc de démontrer que le peptide Cys-Gly dimérisé selon l'invention, agissent en tant qu'agent antioxydant et anti-radicalaire.

Ces conclusions sont, par ailleurs, confortées par l'évaluation de la quantité de protéine carbonylée, un marqueur des dommages provoqués par les radicaux libres ainsi que par l'étude de la péroxydation lipidique. Celle-ci démontre, en effet, que les composés actifs selon l'invention protègent les cellules contres les effets oxydants induits par les UVA. Ces résultats nous permettent de conclure que les peptides selon l'invention activent une série de mécanismes impliqués dans la défense contre le stress cellulaire et l'oxydation.

### Exemple 2 : Mise en évidence de l'effet de l'actif selon l'invention sur la viabilité cellulaire.

### I. Test de cytotoxicité par fixation du rouge neutre.

Des fibroblastes primaires sont ensemencés sur une plaque 96 puits (à 20000 cellules par puits environ). Une fois que les cellules ont atteint 70 % de confluence, le dipeptide dimérisé Cys-Gly selon l'invention, dilué à 1 %, est appliqué sur la plaque durant 24 heures. Une moitié de la plaque est alors irradiée par des UVB à 100 mJ/cm² et l'autre moitié non irradiée. Les cellules sont ensuite remises en culture pendant 24 h en présence de l'actif, puis incubées en présence de rouge neutre.

Le rouge neutre (un colorant « supravital ») se fixe spécifiquement aux lysosomes des cellules vivantes, la coloration sera ainsi proportionnelle à la viabilité des cellules. Après l'ajout d'une solution de révélation, solubilisant le rouge neutre, l'absorbance est mesurée à 540 nm.

Les résultats obtenus, représentés dans le tableau ci-dessous, représentent le pourcentage de viabilité cellulaire en fonction des différentes conditions étudiées, par rapport à la condition contrôle.

| **Conditions d'étude** | **% de viabilité cellulaire** |
|---|---|
| - actif / - UVB | 100 |
| + actif / - UVB | 96,70 |
| - actif / + UVB | 80,00 |
| + actif / + UVB | 94,50 |

Ces résultats nous permettent de conclure que l'actif selon l'invention n'est pas nocif pour les cellules, et bien au contraire, qu'il les protège lorsque celles-ci sont soumises à des agressions d'origines extérieures, notamment les rayonnements UV. On observe, en effet, une augmentation de la viabilité cellulaire de 14,5 % lorsque les cellules sont soumises à une irradiation aux UVB et traitées avec l'actif selon l'invention par comparaison avec les cellules non traitées avec l'actif. Ces résultats démontrent ainsi clairement que le peptide selon l'invention génère un effet protecteur important au niveau cellulaire.

### II. Visualisation et quantification des lésions de l'ADN.

Une étude de l'effet cytoprotecteur de l'actif selon l'invention, a été effectuée en évaluant l'incidence du dipeptide dimérisé Cys-Gly selon l'invention, sur les dommages provoqués au niveau de l'ADN. Cette étude a été réalisée grâce au test des comètes (ou « Single Cell Gel Electrophoresis »), une technique micro-électrophorétique courte et sensible qui permet de visualiser et de quantifier les cassures de l'ADN sur des cellules individuelles.

Une lignée de fibroblastes primaires est ensemencée sur des boîtes de diamètres 100, une fois les cellules arrivées à 70 % de confluence, les échantillons sont préparés et sont traités avec l'actif, dilué à 1 %, 24 heures avant l'irradiation, pendant l'irradiation UVB à 100 mJ/cm², et 24 heures après l'irradiation. Des boîtes non traitées par l'actif servent de contrôle. Une suspension cellulaire à 100 000 cellules/mL est ensuite réalisée, 500 µL sont prélevés, inclus dans une solution d'agarose et coulés entre lame et lamelle. Les cellules sont lysées à froid. L'ADN est ensuite dénaturé par un tampon alcalin suivi d'une courte électrophorèse (250 mA pendant 30 minutes), et il est mis en évidence par ajout de l'iodure de propidium. Les lames sont alors observées au microscope à fluorescence. L'ADN d'une cellule altérée s'étire vers l'anode proportionnellement au nombre de cassures et forme une comète, l'ADN fortement dégradé se retrouve dans la "queue" de la comète. Une cellule intacte reste ronde, l'ADN restant compacté au niveau de la "tête" de la comète.

L'évaluation des lésions de l'ADN s'effectue à l'aide d'un logiciel analyseur d'image qui permet de déterminer le pourcentage de la dégradation d'ADN, par l'évaluation quantitative du «Tail Moment », un paramètre se définissant comme le produit de la longueur de la comète par le pourcentage d'ADN dans sa partie distale.

Le tableau ci-dessous représente l'évaluation des « Tail Moment » mesurés dans des fibroblastes traités suivant les différentes conditions, ainsi que le pourcentage de ce « Tail moment » par rapport au contrôle UV, c'est-à-dire à la condition où les cellules sont soumises à une irradiation sans que l'actif soit appliqué. Le contrôle UV représentant la condition où le « Tail Moment » est le plus important, c'est-à-dire la condition où l'ADN est le plus endommagé.

| **Conditions d'étude** | **Mesure du « Tail Moment »** | **% du « Tail Moment » par rapport au contrôle UV** |
|---|---|---|
| - actif / - UVB | 2648,51 | 2,99 |
| - actif / + UVB | 88592,41 | 100 |
| + actif / - UVB | 1576,82 | 1,78 |
| + actif / + UVB | 23696,54 | 26,75 |

Les résultats obtenus démontrent que les cellules soumises à une irradiation aux UV subissent d'importants dommages au niveau de l'ADN, tandis que les cellules traitées avec l'actif selon l'invention en ont beaucoup moins. En effet, ces résultats démontrent que les cellules traitées avec l'actif ont une protection qui augmente de 73 % lorsque les cellules ont subi une irradiation, par rapport aux cellules non traitées. Ces résultats nous permettent de conclure que le peptide selon l'invention joue un rôle important au niveau de la protection de l'ADN.

### Exemple 3 : Préparation de compositions.

Les quantités indiquées sont données en pourcentage de poids.

### 1- Crème de soin antirides:

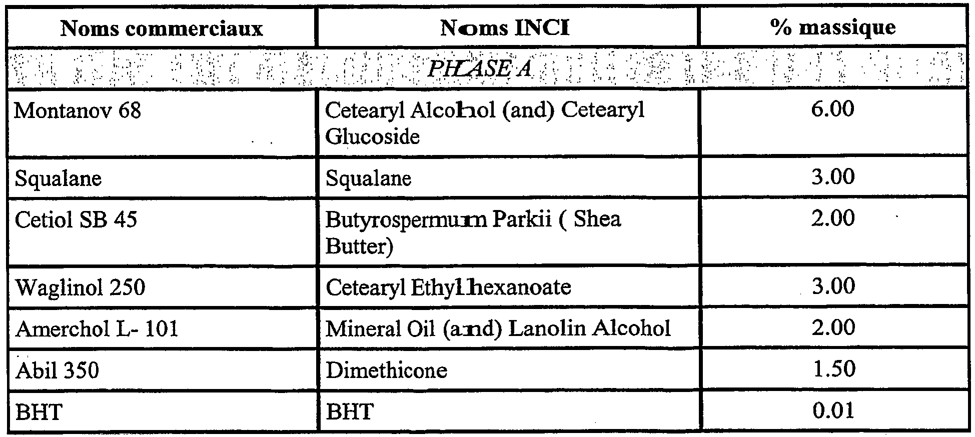

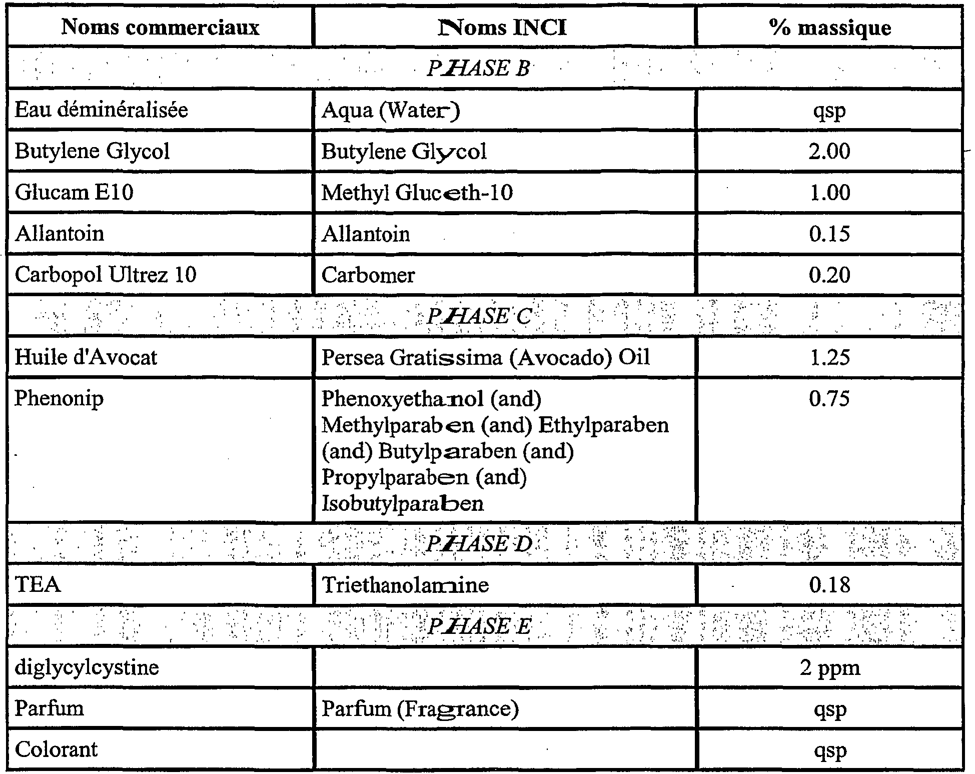

Les constituants de la phase A et de la phase B sont chauffés séparément à une température comprise entre 65°C et 70°C, la phase C est incorporée, puis la phase A est émulsionnée dans la phase B. Le Carbomer est neutralisé avec la phase D à une température aux alentours de 45°C. La phase E est ensuite additionnée sous agitation et le refroidissement est poursuivi jusqu'à 25 °C.

### 2 - Lait corporel anti-âge:

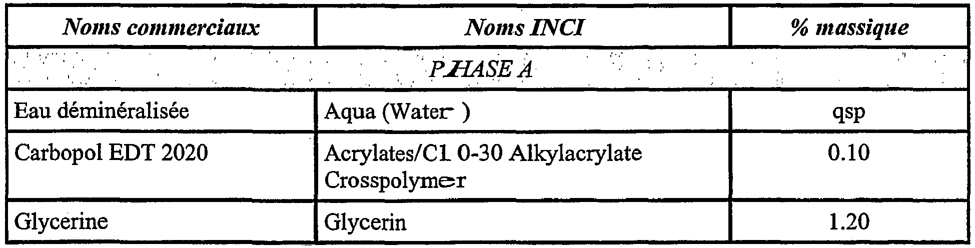

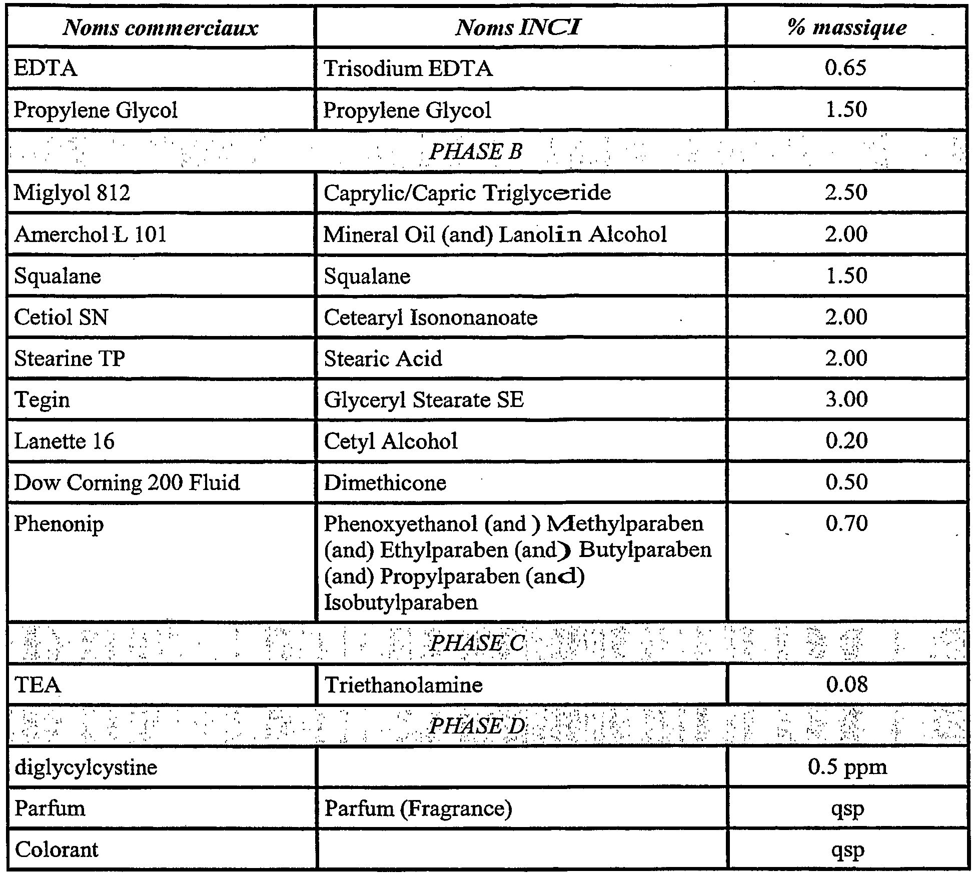

Les constituants de la phase A et de la phase B sont chauffés séparément à une température comprise entre 70°C et 75°C. La phase B est émulsionnée dans A sous agitation « Staro ». Après un refroidissement jusqu'à 50°C, le mélange est neutralisé avec la phase C. La phase D est ensuite additionnée lorsque la température se situe en dessous de 40°C. Le refroidissement est poursuivi jusqu'à 25°C sous agitation lente.

### 3 - Crème protection solaire:

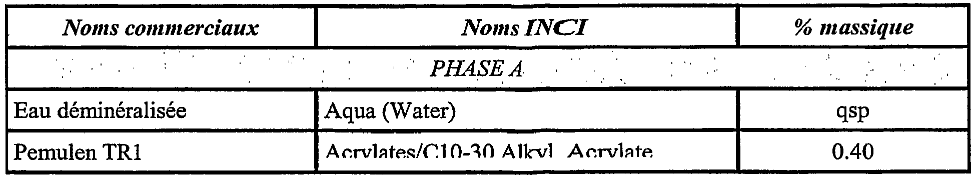

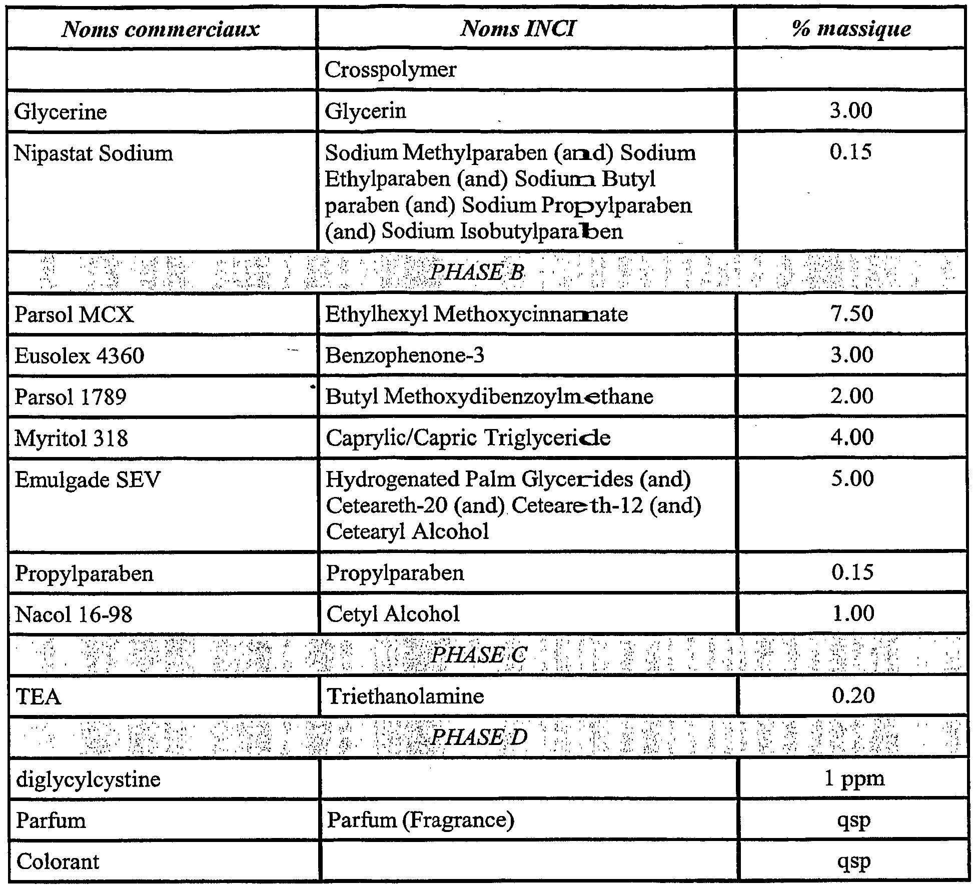

Les constituants de la phase A et de la phase B sont chauffés séparément à une température comprise entre 70°C et 75°C. La phase B est émulsionnée dans A sous agitation. La phase C est ajoutée, à 45°C, en augmentant l'agitation. La phase D est ensuite additionnée lorsque la température se situe en dessous de 40°C. Le refroidissement est poursuivi jusqu'à 25°C sous vive agitation.

## Revendications

1. Utilisation d'une quantité efficace d'au moins un peptide correspondant à la formule générale (I) :
Cys-Gly (I)
en tant qu'agent antioxydant et/ou anti-radicalaire, seul ou en association avec au moins un autre agent actif, dans ou pour la préparation d'une composition cosmétique et/ou dermatologique, **caractérisée en ce que** le peptide est dimérisé par l'intermédiaire d'un pont disulfure.

2. Utilisation selon l'une quelconque des revendications précédentes **caractérisée en ce que** le peptide est choisi parmi les peptides dont au moins un groupement fonctionnel est protégé par un groupement protecteur, ce groupement protecteur étant soit une acylation ou une acétylation de l'extrémité amino-terminale, soit sur une amidation ou une estérification de l'extrémité carboxy-terminale, soit les deux.

3. Utilisation selon l'une quelconque des revendications précédentes **caractérisée en ce que** le peptide est présent dans la composition à une concentration comprise entre 0,005 et 500 ppm environ, préférentiellement à une concentration comprise entre 0,05 et 50 ppm et, encore plus préférentiellement, à une concentration comprise entre 0,1 et 5 ppm.

4. Utilisation selon l'une quelconque des revendications précédentes **caractérisée en ce que** le peptide est préalablement solubilisé dans un ou plusieurs solvants cosmétiquement ou pharmaceutiquement acceptables comme l'eau, l'éthanol, le propylène glycol, le butylène glycol, le dipropylène glycol, les diglycols éthoxylés ou propoxylés, les polyols cycliques, la vaseline, une huile végétale ou tout mélange de ces solvants.

5. Utilisation selon l'une quelconque des revendications précédentes **caractérisée en ce que** le peptide est préalablement solubilisé dans un vecteur cosmétique ou pharmaceutique comme les liposomes ou adsorbés sur des polymères organiques poudreux, des supports minéraux comme les talcs et bentonites, et plus généralement solubilises dans, ou fixés sur, tout vecteur cosmétiquement ou pharmaceutiquement acceptable.

6. Utilisation d'une quantité efficace d'au moins un peptide tel que défini selon l'une quelconque des revendications précédentes, dans ou pour la fabrication d'une composition cosmétique et/ou pharmaceutique, destinée à obtenir une activité protectrice vis-à-vis des espèces réactives de l'oxygène et/ou destinée à prévenir ou traiter les dommages cellulaires provoqués par les radicaux libres.

7. Utilisation d'une quantité efficace d'au moins un peptide tel que défini selon l'une quelconque des revendications 1 à 5, dans ou pour la fabrication d'une composition cosmétique et/ou pharmaceutique destinée à lutter contre les dommages esthétiques provoqués sur la peau et/ou les phanères par les radicaux libres.

8. Utilisation d'une quantité efficace d'au moins un peptide tel que défini selon l'une quelconque des revendications 1 à 5, dans ou pour la fabrication d'une composition cosmétique et/ou pharmaceutique, destinée à lutter de manière préventive et/ou curative contre les manifestations du vieillissement cutané, et tout particulièrement, contre le vieillissement photo-induit.

9. Utilisation d'une quantité efficace d'au moins un peptide tel que défini selon l'une quelconque des revendications 1 à 5, dans ou pour la fabrication d'une composition cosmétique et/ou pharmaceutique, en tant qu'agent photo-protecteur et/ou destinée à protéger la peau et les phanères contre tous les types d'agressions extérieures.

10. Utilisation d'une quantité efficace d'au moins un peptide tel que défini selon l'une quelconque des revendications 1 à 5, dans ou pour la fabrication d'une composition cosmétique et/ou dermatologique, destinée à lutter de manière préventive et/ou curative contre les manifestations cutanées de l'inflammation.

11. Composition cosmétique et/ou dermatologique, comprenant dans un milieu cosmétiquement ou pharmaceutiquement acceptable, au moins un peptide tel que défini selon l'une quelconque des revendications 1 à 5.

12. Composition selon la revendication 11, **caractérisée en ce qu'**elle se présente sous forme d'une solution aqueuse, hydroalcoolique ou huileuse ou sous la forme d'une émulsion huile-dans-eau, eau-dans-huile ou émulsions multiples ou sous forme de crèmes, de suspensions, ou poudres.

13. Procédé de soin cosmétique destiné à lutter contres les dommages inesthétiques provoqués sur la peau et les cheveux par les radicaux libres, induits notamment par les polluants atmosphériques et le rayonnements ultraviolet, **caractérisé par le fait que** l'on applique sur la peau ou les cheveux une composition telle que définie selon l'une quelconque des revendications 11 ou 12.

14. Procédé de soin cosmétique destiné à lutter contre les manifestations cutanées du vieillissement ; et/ou destiné à protéger la peau et les phanères contre les agressions d'origines extérieures ; et/ou destiné à lutter contre l'inflammation cutanée, **caractérisé en ce que** l'on applique sur la peau une composition telle que définie selon l'une quelconque des revendications 11 ou 12.

## Patentansprüche

1. Verwendung einer wirksamen Menge mindestens eines Peptids, das der allgemeinen Formel (I) entspricht:
Cys-Gly (I),
als Antioxidationsmittel und/oder Radikalfänger, allein oder in Verbindung mit mindestens einem anderen Wirkstoff, in der oder zur Herstellung einer kosmetischen und/oder dermatologischen Zusammensetzung, **dadurch gekennzeichnet, dass** das Peptid über eine Disulfidbrücke dimerisiert ist.

2. Verwendung nach einem der vorhergehenden Ansprüche, **dadurch gekennzeichnet, dass** das Peptid aus den Peptiden ausgewählt ist, bei denen mindestens eine funktionelle Gruppe durch eine Schutzgruppe geschützt ist, wobei diese Schutzgruppe entweder eine Acylierung oder eine Acetylierung des aminoterminalen Endes ist, an einer Amidierung oder einer Veresterung des carboxyterminalen Endes oder beides ist.

3. Verwendung nach einem der vorhergehenden Ansprüche, **dadurch gekennzeichnet, dass** das Peptid in der Zusammensetzung in einer Konzentration zwischen 0,005 und ungefähr 500 ppm, vorzugsweise einer Konzentration zwischen 0,05 und 50 ppm und noch mehr bevorzugt einer Konzentration zwischen 0,1 und 5 ppm vorliegt.

4. Verwendung nach einem der vorhergehenden Ansprüche, **dadurch gekennzeichnet, dass** das Peptid vorab in einem oder mehreren kosmetisch oder pharmazeutisch akzeptablen Lösungsmitteln, wie Wasser, Ethanol, Propylenglykol, Butylenglykol, Dipropylenglykol, ethoxylierten oder propoxylierten Diglykolen, cyclischen Polyolen, Vaseline, einem pflanzlichen Öl oder jeglichem Gemisch dieser Lösungsmittel, solubilisiert wird.

5. Verwendung nach einem der vorhergehenden Ansprüche, **dadurch gekennzeichnet, dass** das Peptid vorab in einem kosmetischen oder pharmazeutischen Vektor, wie Liposomen, solubilisiert wird oder auf organischen pulverförmigen Polymeren, mineralischen Trägern, wie Talkum und Bentoniten, adsorbiert wird oder allgemeiner in jeglichem kosmetisch oder pharmazeutisch akzeptablen Vektor solubilisiert wird oder auf diesem fixiert wird.

6. Verwendung einer wirksamen Menge mindestens eines wie in einem der vorhergehenden Ansprüche definierten Peptids in der oder zur Produktion einer kosmetischen und/oder pharmazeutischen Zusammensetzung, die dazu vorgesehen ist, eine Schutzaktivität gegen reaktive Sauerstoffspezies zu erzielen, und/oder dazu vorgesehen ist, durch freie Radikale verursachte Zellschäden zu verhindern oder zu behandeln.

7. Verwendung einer wirksamen Menge mindestens eines wie in einem der Ansprüche 1 bis 5 definierten Peptids in der oder zur Produktion einer kosmetischen und/oder pharmazeutischen Zusammensetzung, die dazu vorgesehen ist, durch freie Radikale an der Haut und/oder den Hautanhangsgebilden verursachte ästhetische Schäden zu bekämpfen.

8. Verwendung einer wirksamen Menge mindestens eines wie in einem der Ansprüche 1 bis 5 definierten Peptids in der oder zur Produktion einer kosmetischen und/oder pharmazeutischen Zusammensetzung, die dazu vorgesehen ist, Manifestationen einer Alterung der Haut und ganz besonders lichtbedingte Alterung auf präventive und/oder heilende Art zu bekämpfen.

9. Verwendung einer wirksamen Menge mindestens eines wie in einem der Ansprüche 1 bis 5 definierten Peptids in der oder zur Produktion einer kosmetischen und/oder pharmazeutischen Zusammensetzung als Lichtschutzmittel und/oder die dazu vorgesehen ist, die Haut und die Hautanhangsgebilde gegen jegliche Arten von äußeren Einflüssen zu schützen.

10. Verwendung einer wirksamen Menge mindestens eines wie in einem der Ansprüche 1 bis 5 definierten Peptids in der oder zur Produktion einer kosmetischen und/oder dermatologischen Zusammensetzung, die dazu vorgesehen ist, Manifestationen einer Entzündung der Haut auf präventive und/oder heilende Art zu bekämpfen.

11. Kosmetische und/oder dermatologische Zusammensetzung, die in einem kosmetisch oder pharmazeutisch akzeptablen Medium mindestens ein wie in einem der Ansprüche 1 bis 5 definiertes Peptid umfasst.

12. Zusammensetzung nach Anspruch 11, **dadurch gekennzeichnet, dass** sie in der Form einer wässrigen, hydroalkoholischen oder öligen Lösung oder in der Form einer Öl-Wasser-Emulsion, Wasser-Öl-Emulsion oder mehreren Emulsionen oder in der Form von Cremes, Suspensionen oder Pulvern vorliegt.

13. Verfahren zur kosmetischen Pflege, die dazu vorgesehen ist, durch freie Radikale an der Haut und den Haaren verursachte, insbesondere durch atmosphärische Schadstoffe und Ultraviolettstrahlung induzierte unästhetische Schäden zu bekämpfen, **dadurch gekennzeichnet, dass** eine wie in einem der Ansprüche 11 oder 12 definierte Zusammensetzung auf die Haut oder die Haare aufgebracht wird.

14. Verfahren zur kosmetischen Pflege, die dazu vorgesehen ist, Manifestationen einer Alterung der Haut zu bekämpfen; und/oder dazu vorgesehen ist, die Haut und die Hautanhangsgebilde gegen Einflüsse äußeren Ursprungs zu schützen; und/oder dazu vorgesehen ist, eine Entzündung der Haut zu bekämpfen, **dadurch gekennzeichnet, dass** eine wie in einem der Ansprüche 11 oder 12 definierte Zusammensetzung auf die Haut aufgebracht wird.

## Claims

1. Use of an effective quantity of at least one peptide corresponding to the general formula (I):
Cys-Gly (I)
as an antioxidant and/or anti-radical agent, alone or in association with at least one further active agent, in or for the preparation of a cosmetic and/or dermatological composition, **characterised in that** the peptide is dimerised by means of a disulphide bond.

2. Use according to any of the above claims **characterised in that** the peptide is chosen from the peptides wherein at least one functional group is protected by a protecting group, this protecting group being either an acylation or an acetylation of the amino-terminus, or an amidation or an esterification of the carboxy-terminus, or both.

3. Use according to any of the above claims **characterised in that** the peptide is present in the composition at a concentration between approximately 0.005 and 500 ppm, preferentially at a concentration between 0.05 and 50 ppm and, more preferentially, at a concentration between 0.1 and 5 ppm.

4. Use according to any of the above claims **characterised in that** the peptide is previously solubilised in one or a plurality of cosmetically or pharmaceutically acceptable solvents such as water, ethanol, propylene glycol, butylene glycol, dipropylene glycol, ethoxylated or propoxylated diglycols, cyclic polyols, petroleum jelly, vegetable oil or any mixture of these solvents.

5. Use according to any of the above claims **characterised in that** the peptide is previously solubilised in a cosmetic or pharmaceutical carrier such as liposomes or adsorbed onto organic polymer powders, mineral substrates such as talcum powders and bentonites, and more generally solubilised in, or bound to, any cosmetically or pharmaceutically acceptable carrier.

6. Use of an effective quantity of at least one peptide as defined according to any of the above claims, in or for the production of a cosmetic and/or pharmaceutical composition, for obtaining a protective activity in respect of reactive oxygen species and/or for preventing or treating cell damage caused by free radicals.

7. Use of an effective quantity of at least one peptide as defined according to any of claims 1 to 5, in or for the production of a cosmetic and/or pharmaceutical composition for combating cosmetic damage caused on the skin and/or skin appendages by free radicals.

8. Use of an effective quantity of at least one peptide as defined according to any of claims 1 to 5, in or for the production of a cosmetic and/or pharmaceutical composition, for combating in a preventative and/or curative manner the signs of skin ageing, and more particularly, photo-induced ageing.

9. Use of an effective quantity of at least one peptide as defined according to any of claims 1 to 5, in or for the production of a cosmetic and/or pharmaceutical composition, as a photoprotective agent and/or for protecting the skin and skin appendages against all types of external attacks.

10. Use of an effective quantity of at least one peptide as defined according to any of claims 1 to 5, in or for the production of a cosmetic and/or dermatological composition, for combating in a preventative and/or curative manner skin signs of inflammation.

11. Cosmetic and/or dermatological composition, comprising in a cosmetically or pharmaceutically acceptable medium, at least one peptide as defined according to any of claims 1 to 5.

12. Composition according to claim 11, **characterised in that** it is presented in the form of an aqueous, hydroalcoholic or oil-based solution or in the form of an oil-in-water, water-in-oil emulsion or multiple emulsions or in the form of creams, suspensions, or powders.

13. Cosmetic treatment method for combating cosmetically unappealing damage caused on the skin and hair by free radicals, induced particularly by atmospheric pollutants and ultraviolet radiation, **characterised in that** a composition as defined according to any of claims 11 or 12 is applied on the skin or hair.

14. Cosmetic treatment method for combating the skin signs of ageing; and/or for protecting the skin and skin appendages against external attacks; and/or for combating skin inflammation, **characterised in that** a composition as defined according to any of claims 11 or 12 is applied on the skin.
